Europäisches Patentamt

European Patent Office

Office européen des brevets

(19)

(11) Publication number: **0 036 712**
A2

# EUROPEAN PATENT APPLICATION

(12)

(21) Application number: 81300808.3

(22) Date of filing: 26.02.81

(51) Int. Cl.³: **C 07 C 67/08**, C 07 C 69/34, C 07 C 69/80, B 01 J 37/02, B 01 J 23/00, B 01 J 31/04

(30) Priority: 01.03.80 GB 8007063

(43) Date of publication of application: 30.09.81 Bulletin 81/39

(84) Designated Contracting States: **BE DE FR GB IT NL**

(71) Applicant: **BP Chemicals Limited, Britannic House Moor Lane, London, EC2Y 9BU (GB)**

(72) Inventor: **Currie, Andrew Christopher, BP CHEMICALS LIMITED Hayes Road Sully, Penarth South Glamorgan CF6 2YU Wales (GB)**
Inventor: **Yeomans, Bertram, BP CHEMICALS LIMITED Saltend Hedon, Hull HU12 8DS (GB)**

(74) Representative: **Harry, John et al, BP INTERNATIONAL LIMITED Patents and Licensing Division Chertsey Road, Sunbury-on-Thames Middlesex TW16 7LN (GB)**

(54) **Heterogeneous esterification catalyst, process for its production and its use in the production of residue esters.**

(57) The invention relates to a catalyst comprising one or more metals having an atomic number greater than or equal to twelve e.g. iron, in filament or particulate form coated with a tin-containing compound such as a carboxylate salt of divalent tin. The catalyst can be prepared by esterifying an alcohol with a carboxylic acid in the presence of the metal and, as esterification catalyst an excess of the tin-containing compound. The catalyst so-prepared is useful, particularly in combination with a homogeneous tin amphoteric catalyst, in the production of high boiling esters.

EP 0 036 712 A2

1

## HETEROGENEOUS ESTERIFICATION CATALYST, PROCESS FOR ITS PRODUCTION AND ITS USE IN THE PRODUCTION OF RESIDUE ESTERS.

The present invention relates to novel heterogeneous esterification catalysts, to a process for their preparation and to their use in a process for the production of high-boiling esters of the type which find use as plasticisers and synthetic lubricants.

Over the years a variety of catalysts have been employed in the production of high-boiling esters, sometimes referred to as residue esters, which include for example phthalates and stearates. Thus strong mineral acids, such as sulphuric acid and phosphoric acid, and organic acids such as para-toluene sulphonic acid have been used extensively. Notwithstanding their extensive use it has long been recognised that problems are associated therewith. Thus in order to achieve commercially viable production rates at temperatures low enough to produce commercially acceptable products it is necessary to employ sufficiently high acid concentrations, which cause dehydration of the alcohol reactant giving rise to unwanted olefins and ethers and colouration of the ester product. The colour may be improved in a subsequent process, either physically, by vacuum distillation of the ester product or chemically by an oxidative treatment to convert the colour-forming impurities to substances which may be recovered by extraction with an aqueous solution of a strong based.

The problems raised by strong acid catalysts are known to be alleviated to a great extent by the use of amphoteric catalysts. These may be described as compounds of elements capable of functioning as cations or anions, as added or subsequently following formation of a complex with the reagents used in the esterification reaction. These include titanium compounds as described for example in British Patent Specifications Nos. 852110, 886750, 1058242, 1061173, 1070914 and 1246346. Typical of such titanium compounds are the titanium esters, e.g. tetraisopropyl titanate. Other amphoteric esterification catalysts are described in British Patent Specification Nos. 879,799 $/\overline{Sb}_2O_3\_7$; 733870 $/\overline{A}l(OH)_3/Na\ OH\_7$; 747260 $/\overline{A}l_2(SO_4)_3/NaOH\_7$; 1076702 $/\overline{Z}nO\ \_7$; 1118363 $/\overline{M}oS_2/C\_7$; 1372854 $/\overline{Z}n(R)_2\_7$ and USP 345729 $/\overline{P}h_3Bi\ \_7$. Another amphoteric esterification catalyst said to be useful in the production of esters having a lower colour intensity than the products obtained by the use of other amphoteric catalysts is stannous oxalate as described in British Patent Specification No. 990297.

A problem associated with the use of some amphoteric catalysts is their lack of solubility in the reaction mixture, it being recognised in the art that only that portion of the catalyst soluble in the reaction mixture is catalytically effective. The kinetic constraint arising from low catalyst solubility and steric hindrance of the catalyst/reagents complex leads to the use of high temperatures, generally up to 220°C, in order to achieve a satisfactory reaction rate. Quite apart from the economic penalty the use of such high temperatures can also lead to product discolouration. We have now developed active heterogeneous esterification catalysts by coating a variety of metals in the form of multifilaments or microspheroids, (i.e. forms of metal having very high surface to volume ratios) with tin-containing compounds.

Thus the present invention provides a heterogeneous catalyst suitable for use in the production of esters which catalyst comprises one or more metals having an atomic number greater than or equal to twelve in filament or particulate form coated with a tin-containing compound.

Suitable metals having an atomic number greater than or equal to 12 may be selected from Groups IB, IIA, IIB, IIIA, IVA, IVB, VA, VB, BIA, VIIA and VIII of the Periodic Table of the Elements as published in the Handbook of Chemistry and Physics, 44th Edition, published by the Chemical Rubber Publishing Co at pages 448/449. Examples of suitable metals are iron, titanium, copper, aluminium and nickel.

Alloys incorporating one or more of the metals may also be employed. Iron is preferably used as the alloy stainless steel, which contains iron as the major component and chromium and nickel as minor components. Metals in filament and particulate form have very high surface to volume ratios. Preferably the metal is in multifilament form. Suitable multifilament forms of the metals copper, titanium, aluminium and stainless steel are commercially available under the trade name "KNITMESH". Such materials typically have a total surface area of approx. 600 sq ft/cu. ft. of the rolled material. Alternatively the metal may be in particulate form, of which microspheroids, e.g. ball bearings , are preferred.

Suitable tin-containing compounds include the carboxylate salts of divalent tin in which the carboxylate group is derived from a monobasic acid, an aliphatic dibasic acid, a tribasic aliphatic acid, a monobasic aromatic acid, a dibasic aromatic acid or a tribasic aromatic acid as hereinafter described in connection with the process for the production of esters. The carboxylate salt may be added as such or may be formed 'in situ' by reacting divalent tin oxide with a carboxylic acid.

According to another aspect of the present invention there is provided a process for the production of a catalyst as hereinbefore described which process comprises contacting at elevated temperature one or more metals having an atomic number greater than or equal to twelve in filament or particulate form with a solution and/or a suspension of a tin-containing compound in an organic solvent capable of dissolving at the elevated temperature from 0.0001 to 1g of the tin-containing compound per 100g of solvent.

Preferably the solvent is one capable of dissolving from 0.001 to 0.1g of tin-containing compound per 100g of solvent at the temperature of the reaction. Suitable solvents include esters, ketones and alcohols.

The elevated temperature may suitably be greater than 100°C and is preferably in the range from 150 to 300°C.

A very convenient method for preparing the catalyst comprises carrying out an esterification process in the presence of the metal, in filament or particulate form, employing as catalyst an excess of a carboxylate salt of divalent tin capable of functioning as a homogeneous esterification catalyst.

Suitably in the preparation of the coating the amount of homogeneous catalyst added may be greater than 1g, preferably from 3 to 100g of tin metal ion per square metre of surface area of metal, i.e. metal in filament or particulate form plus metal, if any, forming the surface of the reactor in which the esterification process is carried out.

After carrying out the esterification thereby coating the metal with tin compound any excess homogeneous catalyst may be removed with the ester product.

The present invention also provides a process for the production of esters boiling above 100°C which process comprises reacting an alcohol with a carboxylic acid or a carboxylic acid anhydride at a temperature greater than 160°C in the presence of a heterogeneous esterification catalyst as hereinbefore described.

The process of the invention is generally applicable to esters which boil above 100°C at atmospheric pressure. It is particularly applicable to esters derived from the following acids:-

monobasic acids - containing up to 20 carbon atoms, e.g. alkanoic acids such as myristic, palmitic and stearic acid, alkenoic acids such as oleic acid or derivatives of such alkanoic and alkenoic acids, e.g. ricinoleic acid;

aliphatic dibasic acids - especially those containing up to 20 carbon atoms, preferably up to 10 carbon atoms such as adipic, azelaic or sebacic acid;

tribasic aliphatic acids - such as citric acid;

monobasic aromatic acids - suitably those containing up to 10 carbon atoms, such as benzoic acid.

dibasic aromatic acids and their anhydrides - such as the three phthalic acids especially phthalic acid itself (ortho-phthalic acid) or phthalic anhydride, and their hydrogenation products, e.g. hexahydrophthalic anhydride, and

tribasic aromatic acids and their anhydrides - such as hemimellitic, trimellitic or trimesic acids and their anhydrides.

The preferred acids are ortho-phthalic acid or its anhydride, adipic acid, azelaic acid or sebacic acid.

The process of the invention is particularly applicable to esters derived from the following alcohols:-

monohydric alcohols - containing up to 20 carbon atoms, particularly alkanols containing from 4 to 14 carbon atoms, e.g. butanol, isoheptanol, iso-octanol, 2-ethylhexanol, nonanol, decanol, tridecanol or mixtures of alcohols containing, for example, 7 to 9 carbon atoms such as are obtained from olefinic mixtures by the OXO process;

dihydric alcohols - containing up to 20 carbon atoms, e.g. monoethylene glycol, diethylene glycol, triethylene glycol, mono-, di- or tripropylene glycol, the butylene glycols or 2,2,4,-trimethylpentane diol;

trihydric alcohols - such as glycerol;

aliphatic cyclic alcohols - containing up to 12 carbon atoms such as cyclohexanol;

derivatives - particularly other derivatives of the dihydric and trihydric alcohols, e.g. lower alkyl ether derivatives such as 2-butoxy ethanol.

The preferred alcohols are the monohydric alcohols containing 4 to 14 carbon atoms.

Preferably there is also added to the esterification reaction mixture an amphoteric tin catalyst which is soluble in the mixture. Suitably this catalyst is a carboxylate salt of divalent tin. The amount of soluble tin catalyst added may suitably be greater than 0.005% and preferably between 0.01 and 1.0% by weight of the reaction mixture. It is believed to be highly desirable for optimum results to employ a soluble tin catalyst in addition to the heterogeneous catalyst because it is thought that an equilibrium exists between the tin-containing coating and the reaction medium.

Preferably the esterification reaction temperature is in the range from 180 to 225°C. The temperature may suitably be controlled by the addition of an inert diluent of appropriate boiling point and/or by the use of sub-atmospheric pressure. Suitable inert diluents include hydrocarbons such as toluene or ortho-xylene, of which ortho-xylene is preferred. The amount of inert diluent added may suitably be greater than 5% w/w, preferably from 15 to 30% w/w, based on the weight of the reaction mixture.

In order to take the esterification reaction to completion the amount of alcohol added should be in excess of the stoichiometric amount required to react completely with the acid or anhydride and the water of reaction removed as an azeotrope with excess alcohol, which may be separated and returned to the reaction. Up to a 50% stoichiometric excess, preferably from 10 to 30% stoichiometric excess may be employed. Alternatively or in addition the water of reaction may be removed by the addition of an entrainer which may suitably be the hydrocarbon diluent.

Upon completion of the esterification reaction any unconverted alcohol present in the ester product may be removed by steam-stripping. Alcohol collected in this manner is preferably recycled to the reaction.

It is preferred to remove any residual acidity in the ester product and to remove any esterification catalyst present therein. This may suitably be achieved by the addition of an inorganic strong base, such as sodium carbonate or lime, which may be added either in the form of a solid or as an aqueous solution. Procedures and conditions for carrying out this step are well-known in the art and typically involve the use of elevated temperature.

The steps of alcohol stripping and neutralisation/catalyst removal are preferably effected outside the reactor in which the esterification process is carried out because the conditions and/or reagents employed have a detrimental effect on the tin coated metal catalyst. It is therefore preferred to carry out the esterification process in a batch reactor and effect the operations on the ester product batchwise in a separate vessel. The batch reactor in which the esterification process is carried out may be fabricated in any suitable material such as stainless steel, titanium or glass. Preferably the reactor is fabricated in stainless steel.

The esterification process forming one aspect of the present invention is particularly applicable to the production of plasticisers for use in the plastics industry from for example phthalic anhydride and a $C_4$ to $C_{14}$ alkanol or mixture of such alkanols and for the production of esters which are synthetic lubricants.

The invention will now be described by reference to the following Examples:

Example 1

Method of coating metal surface with stannous oxalate to form an esterification catalyst.

A mixture of phthalic anhydride (370 grams) PA and 2-ethylhexanol (781 grams) was added to a 2 1 cylindrical, 845-Ti, stainless steel reactor lined with 316 L stainless steel "KNITMESH" (viz. two rolls. 10 cm x 5 cm diameter; surface area 4 ft.$^2$) together with 10 grams of stannous oxalate. The charge was heated to 190°C with stirring and nitrogen sparging was maintained throughout the heating up and reaction periods.

Water was removed from the reactor during the course of the reaction via a Dean and Stark adapter, overhead reflux being maintained by reducing the pressure in the system from 0.9 to 0.2 bar during the course of the reaction. The rate of the reaction was followed by monitoring the change in acidity of the reaction mixture.

A 99.97% conversion of PA to ester was achieved after 3h. The reaction mixture was subsequently cooled to ambient temperature prior to discharging the reactor contents, the "KNITMESH" packing being washed several times with the crude ester product to remove any trapped suspension of excess metal salt. 69% of the catalyst addition formed the catalyst coating. An investigation of the coating by electron spectroscopy chemical analysis (ESCA) indicated the presence of up to 200 Angstrom thickness of tin metal ion together with oxide and carboxyl groups.

Example 2

Esterification catalysed by stannous oxalate-coated metal.

Esterification of phthalic anhydride (2.5 moles) and 2-ethylhexanol (6 moles) was carried out at 225°C in a glass reactor containing 316 stainless steel "KNITMESH" (two 10 cm x 5 cm diameter rolls) which had previously been coated with ca. 6g of stannous oxalate by the procedure described in Example 1. No catalyst addition apart from the tin-coated "KNITMESH" was made. A 99.8% conversion of phthalic anhydride to dioctyl phthalate was achieved after 5 hours.

Comparison Test

Example 2 was repeated except that the stannous oxalate-coated stainless steel "KNITMESH" was omitted. A 99.8% conversion of phthalic anhydride to ester was achieved after a reaction period of 40 h.

This is not an example according to the invention and is included only for the purpose of comparison.

The results of Example 2 and the Comparison Test demonstrate that stannous oxalate-coated stainless steel "KNITMESH" is an effective catalyst for the production of dioctyl phthalate, there being a significant reduction in batch esterification time from 40 to 5 hours.

0036712

Claims

1. A catalyst suitable for use in the production of esters which catalyst comprises one or more metals having an atomic number greater than or equal to 12 in filament or particulate form coated with a tin-containing compound.

2. A catalyst according to claim 1 wherein the metal is iron, titanium, copper, aluminium or nickel.

3. A catalyst according to either claim 1 or claim 2 wherein the metal is in multifilament form.

4. A catalyst according to any one of the preceding claims wherein the tin-containing compound is a carboxylate salt of divalent tin.

5. A process for the production of a catalyst as claimed in any one of claims 1 to 4 which process comprises contacting at elevated temperature one or more metals having an atomic number greater than or equal to twelve in filament or particulate form with a solution and/or a suspension of a tin-containing compound in an organic solvent capable of dissolving at the elevated temperature from 0.0001 to 1g of the tin-containing compound per 100g of solvent.

6. A process according to claim wherein an esterification process is carried out in the presence of the metal employing as catalyst an excess of a carboxylate salt of divalent tin capable of functioning as a homogeneous esterification catalyst.

7. A process according to claim 5 wherein the carboxylate salt of divalent tin is formed 'in situ' by reacting divalent tin oxide with the carboxylic acid.

8. A process according to either claim 6 or claim 7 wherein the amount of homogeneous esterification catalyst added is from 3 to 100g of tin metal ion per square metre of metal surface area.

9. A process for the production of esters boiling above 100°C which process comprises reacting an alcohol with a carboxylic acid or a carboxylic acid anhydride at a temperature greater than 160°C in the presence of the heterogeneous esterification catalyst as claimed in any one of claims 1 to 4.

10. A process according to claim 9 wherein the carboxylic acid is ortho-phthalic acid, adipic acid, azelaic acid or sebacic acid.

11. A process according to either claim 9 or claim 10 wherein the alcohol is a monohydric alcohol containing from 4 to 14 carbon atoms.

12. A process according to any one of claims 9 to 11 wherein there is also added to the esterification reaction mixture an amphoteric tin catalyst which is soluble in the mixture.

13. A process according to claim 12 wherein the soluble amphoteric tin catalyst is a carboxylate salt of divalent tin.

14. A process according to either claim 12 or claim 13 wherein the amount of soluble tin catalyst added is between 0.01 and 1.0% by weight of the reaction mixture.

15. A process according to any one of claims 9 to 14 wherein the temperature is in the range from 180 to 225°C.